Europäisches Patentamt

European Patent Office

Office européen des brevets

(19)

(11) **EP 0 561 090 B1**

(12) **FASCICULE DE BREVET EUROPEEN**

(45) Date de publication et mention
de la délivrance du brevet:
**04.06.1997 Bulletin 1997/23**

(51) Int Cl.⁶: **C12P 19/16**, C12P 19/14,
C12P 19/04, A23L 1/308,
A61K 31/715

(21) Numéro de dépôt: **92403561.1**

(22) Date de dépôt: **28.12.1992**

(54) **Procédé de préparation de polysaccharides peu digestibles, éventuellement hydrogénés**

Verfahren zur Herstellung von schwerverdaubaren Polysacchariden, die gegebenenfalls hydriert sind

Process for the preparation of not easily digestible possibly hydrogenated polysaccharides

(84) Etats contractants désignés:
**AT BE CH DE DK ES FR GB GR IE IT LI LU MC NL
PT SE**

(30) Priorité: **19.03.1992 FR 9203313**

(43) Date de publication de la demande:
**22.09.1993 Bulletin 1993/38**

(73) Titulaire: **Roquette Frères
F-62136 Lestrem (FR)**

(72) Inventeur: **Caboche, Jean-Jacques
F-62400 Bethune (FR)**

(74) Mandataire: **Boulinguiez, Didier et al
Cabinet Plasseraud
84, rue d'Amsterdam
75009 Paris (FR)**

(56) Documents cités:
**EP-A- 0 063 909          EP-A- 0 257 535
EP-A- 0 368 451          WO-A-92/02614**

## Description

La présente invention concerne un procédé de préparation de polysaccharides peu digestibles et des produits hydrogénés correspondants. Elle a également pour objet les produits susceptibles d'être obtenus à l'aide de ce procédé.

Au cours de la dernière décennie, un intérêt de plus en plus grand s'est porté vers la consommation de fibres alimentaires ou de produits ayant un effet analogue à celui des fibres alimentaires, afin d'améliorer le transit intestinal surtout chez les habitants des pays hautement développés pour lesquels les produits alimentaires sont de plus en plus raffinés et contiennent donc de moins en moins de fibres naturelles.

La consommation quotidienne, ou quasi quotidienne de fibres alimentaires, est également réputée diminuer la fréquence des cancers du colon.

Ceci étant, l'obtention de fibres alimentaires extrêmement raffinées ou extrêmement pures n'est pas toujours aisée à réaliser et les fibres insolubles obtenues à partir de produits végétaux peuvent parfois avoir un effet contraire, c'est-à-dire provoquer l'irritation du colon et par là-même avoir des effets néfastes sur les muqueuses intestinales.

L'attention s'est donc portée de plus en plus vers l'obtention de fibres solubles. Une des nombreuses voies de recherche dans ce domaine a été d'obtenir lesdites fibres solubles par hydrolyse de matières amylacées ou par réarrangement de produits issus desdites matières amylacées.

Ainsi par exemple, un produit dénommé communément polydextrose a été fabriqué il y a une vingtaine d'années, ce produit étant obtenu par réarrangement de molécules de glucose grâce à un chauffage dans des conditions relativement anhydres en présence d'acides alimentaires, une petite quantité de sorbitol étant également présente dans le milieu réactionnel.

Ce polydextrose, réputé d'une valeur calorique d'environ une calorie par gramme, souffre cependant de beaucoup de désavantages notamment en raison de son goût relativement peu agréable et de sa tendance à la coloration; par ailleurs il ne présente pas toutes les qualités requises pour une fibre alimentaire.

Ce produit, compte tenu de son procédé de fabrication, constitue en fait un mélange mal défini de produits très différents et de masses moléculaires très diverses. Il est ainsi caractérisé le plus généralement par une masse moléculaire moyenne ne reflétant qu'imparfaitement la masse moléculaire de toutes les molécules entrant dans sa composition. La présence dans ce produit de composés amers et acides limite fortement ses applications.

Un autre procédé déjà décrit dans la littérature a consisté à transformer l'amidon en une dextrine comportant des liaisons différentes de celles trouvées dans l'amidon d'origine, puis à procéder à une hydrolyse de cette dextrine à l'aide d'une α-amylase, cette action de l'α-amylase étant éventuellement complétée par l'action d'autres enzymes. Une telle démarche a ainsi par exemple été décrite dans la demande de brevet EP n° 0 368 451.

L'un des avantages essentiels du procédé décrit dans la susdite demande de brevet est de pouvoir, grâce à l'action enzymatique de l'α-amylase, éliminer ou réduire très notablement le goût et l'odeur désagréables de la dextrine de départ. Cependant, les produits obtenus selon ce procédé ne sont pas aussi peu digestibles que l'on pourrait le souhaiter. Leur qualité en tant que fibres alimentaires solubles est donc loin d'être optimale.

La présente invention a pour objet de fournir un procédé permettant de fabriquer très simplement, à partir de dextrines obtenues à partir d'amidon, des fibres alimentaires solubles beaucoup moins digestibles que toutes celles proposées dans l'art antérieur et qui permettent par là-même d'obtenir un effet de fibres alimentaires tout à fait optimal.

Le procédé de préparation de polysaccharides peu digestibles conforme à l'invention est caractérisé par le fait qu'il comprend l'hydrolyse enzymatique d'au moins une dextrine et/ou un polyglucose à l'aide d'au moins une enzyme saccharifiante et d'au moins une enzyme hydrolysant les liaisons 1-6 de l'amylopectine.

C'est essentiellement grâce à l'action de l'enzyme hydrolysant les liaisons 1-6 de l'amylopectine que les polysaccharides obtenus à l'issue de ce procédé présentent une digestibilité minimale.

De façon inattendue, il a été constaté de plus que le procédé de préparation de polysaccharides peu digestibles, conforme à l'invention, permet également d'obtenir un produit ayant d'excellentes qualités organoleptiques, ceci sans avoir recours nécessairement à l'emploi d'α-amylase comme préconisé dans l'art antérieur. Le produit obtenu est particulièrement neutre en goût, sans couleur, sans odeur et sans amertume. Enfin, il présente une très grande stabilité aux enzymes de microorganismes.

On peut préparer les polysaccharides peu digestibles selon le procédé conforme à l'invention en s'y prenant comme suit ou de manière équivalente.

Une dextrine obtenue à partir d'amidon est dispersée ou solubilisée dans l'eau à une matière sèche généralement comprise entre 20 et 70 % et de préférence comprise entre 20 et 45 %.

La suspension ainsi obtenue est alors soumise à un traitement enzymatique comportant au moins l'action d'une enzyme saccharifiante et d'une enzyme hydrolysant les liaisons 1-6 de l'amylopectine, les conditions de ce traitement enzymatique étant choisies de façon telle que le DE (Dextrose Equivalent) de l'hydrolysat obtenu soit compris entre 5 et 80, de préférence entre 7 et 60, et plus préférentiellement encore entre 10 et 50.

Ce procédé permet également de préparer des polysaccharides peu digestibles à partir de polyglucose ou de mélanges de dextrine et de polyglucose.

L'hydrolyse enzymatique de polyglucoses, en particulier du polydextrose, a déjà été réalisée, mais par des glucoamylases tels que l'α-amylase, l'isomaltodextranase, la glucodextranase et la glucoamylase, et ceci dans un but purement analytique visant à déterminer la structure particulière du polydextrose. Citons notamment les travaux à ce sujet de Kobayashi T. et Yoshino H. (Denpun Kagaku, 1989, 36 (4), 283-6 (Japan)).

A la connaissance de la demanderesse, il n'avait jamais été envisagé jusqu'à ce jour d'hydrolyser des polyglucoses à l'aide d'enzymes hydrolysant les liaisons 1-6 de l'amylopectine tels que l'isoamylase et la pullulanase, et de préparer ainsi des polysaccharides ayant un effet de fibres alimentaires tout à fait optimal.

Par le terme "polyglucose", au sens de la présente invention, on entend les produits composés majoritairement de liaisons 1-6, obtenus par condensation ou réarrangement, à partir du glucose ou d'un ou plusieurs sucres éventuellement réduits, sous l'action combinée de la chaleur et d'acides dans un milieu presque dépourvu d'eau. De tels polymères ont été décrits de nombreuses fois et peuvent être obtenus par des procédés tels que ceux décrits notamment dans les brevets US 2 436 967, US 3 766 165, US 4 965 354, US 5 051 500, JP 01-12761 et JP 02-163101. De façon avantageuse, ces polymères sont obtenus à partir de glucose et d'acide citrique, éventuellement en présence de sorbitol, tels que les produits de type polydextrose susmentionné.

Par le terme "dextrine" on entend les produits obtenus par chauffage de l'amidon amené à un faible taux d'humidité, en présence généralement de catalyseurs acides ou basiques. Ce "grillage à sec" de l'amidon, le plus couramment en présence d'acide, entraîne à la fois une dépolymérisation de l'amidon et un réarrangement des fragments d'amidon obtenus, conduisant à l'obtention de molécules très ramifiées.

Les dextrines font partie des dérivés d'amidon les plus anciens et leur préparation, leurs applications, les différents types de dextrines ainsi que leurs propriétés sont décrits par exemple dans l'ouvrage intitulé "Starch Chemistry and Technology" - Second Edition - Edited by Roy L. WHISTLER - 1984 - Academic Press Inc.

De préférence, on utilise pour la préparation des polysaccharides conformes à l'invention des dextrines obtenues par grillage à sec de l'amidon en présence d'un catalyseur acide comme l'acide chlorhydrique. L'acide, sous forme de solution diluée, est ainsi pulvérisé sur l'amidon et le mélange obtenu est préséché, par exemple de 80 à 130°C jusqu'à une teneur en eau inférieure ou égale à environ 5 %. Puis le mélange est "grillé" à une température d'environ 140°C à 250°C pendant une durée de 30 minutes à environ 6 heures pour obtenir la dextrine, qui présente en fin de réaction un DE de 0,5 à 10 environ. On peut utiliser, pour la préparation de ces dextrines, n'importe quel type d'amidon, et notamment l'amidon de maïs, la fécule de pomme de terre, l'amidon de blé, la fécule de manioc, l'amidon de riz ou l'amidon de pois.

D'après la norme ISO 1227 de 1979, une dextrine est obtenue à partir d'amidon ou de fécule transformée par chauffage à sec avec ou sans addition de petites quantités de réactifs chimiques. Traditionnellement, les dextrines sont classés en deux catégories : les dextrines blanches dont l'aspect est peu différent de celui de la matière première utilisée, et les dextrines jaunes, produites dans des conditions plus drastiques et dont l'intensité de la coloration peut être corrélée au degré de modification de la structure native. Les quatre types de réaction intervenant lors de la dextrinification sont, aux faibles températures, essentiellement l'hydrolyse des liaisons alpha 1-4, puis, aux températures plus élevées, des réactions de condensation de transglycosidation entraînant un réarrangement, et enfin d'anhydrisation.

Des dextrines telles que celles commercialisées par la Société Demanderesse sous les marques TACKIDEX DF 165, TACKIDEX DF 155, TACKIDEX JO 55 K peuvent être avantageusement utilisées.

L'hydrolysat obtenu à la suite de la saccharification et de l'action de l'enzyme hydrolysant les liaisons 1-6 de l'amylopectine est ensuite purifié de manière connue en soi par décoloration sur charbon actif et déminéralisation sur résines échangeuses d'ions. Les hydrolysats peuvent également être décolorés à l'aide d'agents oxydants ou réducteurs.

Le produit obtenu peut alors être éventuellement hydrogéné, toujours de manière connue en soi, par exemple sur catalyseur au nickel de Raney ou sur catalyseurs aux métaux nobles.

De la sorte, on obtient un produit stable encore à la cuisson et au stockage, présentant une réactivité chimique moindre et également la propriété intéressante de n'être pas cariogène.

L'enzyme saccharifiante utilisée conformément au procédé de la présente invention est de préférence choisie parmi la β-amylase et l'amyloglucosidase, ces deux enzymes pouvant cependant être utilisées de façon simultanée ou successive.

De préférence, les quantités et conditions d'action de ces différentes enzymes saccharifiantes pouvant être employées pour la préparation des polysaccharides peu digestibles conforme à l'invention sont choisies parmi les suivantes :

- β-amylase : 1,45 à 145 unités internationales (100 à 10 000 unités LINTNER) par kg de substrat sec, température de 50°C à 60°C, durée d'action de 30 à 100 heures, pH de 5,0 à 6,0
- amyloglucosidase : 4 000 à 500 000 unités internationales par kg de substrat sec, température de 50°C à 60°C,

EP 0 561 090 B1

durée d'action de 30 à 100 heures, pH de 5.0 à 6.0.

En ce qui concerne les enzymes hydrolysant les liaisons 1-6 de l'amylopectine, elles sont choisies parmi la pullulanase et l'isoamylase, des enzymes commerciales comme la PULLUZYME 750 L de la Société ABM ou la CK 20 L de la Société AMANO ou la PROMOZYME de la Société NOVO pouvant ainsi être utilisées. Les quantités et conditions d'action de ces enzymes sont les suivantes :

435 à 43500 unités internationales [150 à 15 000 unités ABM (Société ABM, CHESHIRE, ENGLAND)] par kg de substrat sec, pH de 5,0 à 6,0, température de 50°C à 60°C, durée d'action de 24 à 100 heures.

L'hydrolyse de la dextrine et/ou de polyglucose à l'aide d'une enzyme saccharifiante et d'une enzyme hydrolysant les liaisons 1-6 de l'amylopectine peut éventuellement être précédée, accompagnée ou suivie d'une action complémentaire à l'aide d'une $\alpha$-amylase ou peut éventuellement être précédée d'une hydrolyse acide.

En ce qui concerne cette dernière éventualité, on peut signaler qu'il n'est pas forcément nécessaire d'ajouter une quantité d'acide à la dextrine, cette dernière comportant à l'issue de l'étape de dextrinification proprement dite une quantité résiduelle d'acide suffisante pour assurer l'hydrolyse acide de l'amidon insoluble ou imparfaitement dextrinifié.

De ce fait, l'action d'une $\alpha$-amylase préalablement à l'action enzymatique des enzymes saccharifiantes et des enzymes hydrolysant les liaisons 1-6 de l'amylopectine n'est généralement pas nécessaire.

Par contre, l'action d'une $\alpha$-amylase peut parfois s'avérer intéressante à la suite de l'action de l'enzyme saccharifiante et de l'enzyme hydrolysant les liaisons alpha 1-6.

Au cas où de l'$\alpha$-amylase est utilisée dans le procédé conforme à l'invention, on utilise de préférence des $\alpha$-amylases bactériennes ou fongiques, et les conditions et les quantités d'action de l'$\alpha$-amylase sont généralement les suivantes:

- 0,35 à 35 unités internationales [20 à 2 000 unités KNU (Kilo Novo Units)] par kg de substrat sec, pH de 5,0 à 6,0, température de 50°C à 60°C, durée d'action de 16 à 100 heures.

L'hydrogénation du produit obtenu à la suite de l'hydrolyse enzymatique de la dextrine et/ou de polyglucose peut être effectuée de manière connue en soi par hydrogénation sur Nickel de Raney ou par hydrogénation sur métaux nobles. Cette hydrogénation est effectuée après purification du produit, par exemple par traitement sur charbon actif, suivi d'une déminéralisation sur résines cationiques et anioniques. L'hydrogénation peut être effectuée par exemple sur catalyseur au Nickel de Raney, à une température de 130°C et sous une pression d'hydrogène de 50 bars.

Après hydrogénation, le produit hydrogéné obtenu est filtré, déminéralisé, puis concentré jusqu'à la concentration de commercialisation qui est généralement comprise entre 40 et 70 Brix environ. On rappelle que le Brix est une unité de mesure couramment employée dans l'industrie amidonnière, et que le Brix d'un sirop est déterminé très aisément par lecture au réfractomètre. Un Brix d'environ 75 correspond pour les produits visés par l'invention à une matière sèche d'environ 70 %.

L'hydrogénation est conduite jusqu'à l'obtention d'un pourcentage de sucres réducteurs résiduels sur matière sèche inférieure à 2,0, de préférence inférieure à 1,0 et plus préférentiellement encore inférieure à 0,25.

Les produits obtenus à la suite de l'hydrolyse enzymatique de la dextrine et/ou de polyglucose, qu'ils soient hydrogénés ou non, peuvent également être présentés sous forme pulvérulente, cette forme pulvérulente pouvant être obtenue par exemple par atomisation.

On peut parfois souhaiter augmenter la richesse en polysaccharides peu digestibles du produit obtenu à l'issue de l'hydrolyse enzymatique de la dextrine et/ou du polyglucose et de l'hydrogénation éventuelle en éliminant au maximum les molécules de faible poids moléculaire présentes dans lesdits produits, ces molécules pouvant être constituées par exemple de glucose, de maltose ou d'oligosaccharides de faible poids moléculaire, ou de leurs éventuels homologues hydrogénés.

Ces molécules de faible poids moléculaire peuvent être éliminées par exemple par séparation chromatographique sur résines cationiques, mises sous forme alcaline ou alcalino-terreuse, ou sur zéolites, ou elles peuvent être éliminées par des procédés tels que les techniques membranaires comme l'ultrafiltration ou l'osmose inverse ou par précipitation à l'aide de solvants comme par exemple les alcools.

Grâce à l'action prévue dans le cadre de la présente invention d'une enzyme hydrolysant les liaisons 1-6 de l'amylopectine, on obtient des polysaccharides beaucoup moins digestibles que les polysaccharides obtenus à la suite d'une hydrolyse enzymatique ne comportant pas l'action supplémentaire de cette enzyme.

Afin de caractériser la digestibilité des polysaccharides obtenus à l'aide du procédé conforme à l'invention, on peut déterminer la teneur en polysaccharides non digestibles par l'amyloglucosidase dans un test F.

Ce test correspond au test de détermination des "fibres alimentaires totales" développé par la Société SIGMA Chemical Company, P.O. Box 14508, St Louis, M.O. 63178 USA. Il est décrit dans le bulletin technique SIGMA No TDFAB-A de juin 1991.

Ce test consiste essentiellement à déterminer la quantité de matière contenue dans l'hydrolysat, non hydrolysable par une amyloglucosidase en présence d'une α-amylase thermorésistante et d'une protéase. Cette quantité est exprimée en pourcentage par rapport à une quantité de 1 g environ d'hydrolysat préalablement séché sous vide à 70°C pendant une nuit.

Pour conduire ce test, on procède comme suit :

1) On pèse à 0,1 mg près quatre échantillons de 1 g environ d'hydrolysat préalablement séché sous vide et refroidi dans un dessicateur pendant 1 nuit, que l'on introduit dans un bécher forme haute de 400 ml.

2) On ajoute dans chacun des quatre béchers 50 ml d'un tampon phosphate (0,05 M) à pH : 6,0.

3) On ajoute 0,05 ml d'une solution d'alpha-amylase (produit Sigma n° A 3306) dans chacun des béchers, et l'on mélange intimement.

4) On couvre chaque bécher avec un film aluminium avant de les placer dans un bain d'eau bouillante pour les incuber pendant 30 mn à partir du moment où la température atteint dans les béchers 95°C. On agite doucement à intervalles réguliers de 5 minutes.

5) On refroidit les solutions à température ambiante.

6) On ajuste le pH des solutions à 7,5 ± 0,1 par ajout dans chaque bécher de 10 ml de NaOH 0,171N. On vérifie le pH et on l'ajuste éventuellement avec de la soude (0,171 N) ou de l'acide phosphorique (0,205 M).

7) On ajoute 5 mg de protéase en poudre (produit Sigma n° P-3910) à chacun des béchers.

8) On couvre les béchers avec un film d'aluminium et on les incube à 60°C pendant 30 mn sous agitation continue. Le temps d'incubation de 30 mn débute à partir du moment où la température interne des béchers atteint 60°C.

9) On refroidit à température ambiante.

10) On ajoute 10 ml d'$H_3PO_4$ 0,205 M à chacun des béchers pour ajuster le pH à 4,5 ± 0,2. On vérifie le pH. On ajuste éventuellement avec précaution avec les solutions de soude ou d'acide phosphorique.

11) On ajoute 0,3 ml d'amyloglucosidase (produit Sigma N A.9913) à chaque bécher.

12) On couvre chacun des béchers avec un film d'aluminium et on incube pendant 30 minutes à 60°C sous agitation continue. Le temps d'incubation de 30 mn débute à partir du moment où la température interne des béchers atteint 60°C.

13) On ajoute 280 ml d'éthanol à 95 % (v/v), préchauffé à 60°C, à chacun des béchers. (éthanol à 95 % v/v : 50 ml d'eau déminéralisée, éthanol absolu q.s.p. 1000 ml à 20°C).

14) On laisse se former un précipité par abandon à température ambiante pendant au moins 60 minutes, ou une nuit (même temps pour chacun des 4 essais).

15) On filtre sous vide, sur creuset de verre fritté et lit de Celite le contenu de chacun des béchers qu'on lave successivement et avec précaution avec :

- trois fois 20 ml d'éthanol à 78 % (v/v) (éthanol à 78 % v/v : 220 ml d'eau déminéralisée, éthanol absolu q.s.p. 1000 ml à 20°C)
- deux fois 10 ml d'éthanol à 95 % (v/v)
- et deux fois 10 ml d'acétone.

16) On sèche les quatre filtres à 70°C sous vide pendant 1 nuit.

17) On refroidit ces filtres dans un dessicateur avant de les peser à 0,1 mg près, considérant ce poids comme la somme du poids de résidu de filtration (polysaccharides non hydrolysables à l'amyloglucosidase, plus protéines plus cendres) et du poids du creuset avec Celite.

18) On détermine les teneurs en protéines de deux des quatre résidus de filtration provenant des quatre essais en procédant selon la méthode Kjeldahl en utilisant le facteur de correction de 6,25.

19) On détermine les quantités de cendres sur les deux autres résidus de filtration en plaçant les creusets dans un four à 525°C pendant 5 heures.

20) On calcule, comme indiqué dans le Bulletin technique SIGMA, les quantités de polysaccharides non hydrolysables à l'amyloglucosidase pour les quatre essais et on réalise une moyenne de ces quantités que l'on ramène à la moyenne des quantités de matière d'hydrolysat séché à 70°C sous vide pendant une nuit, en tenant compte dans le calcul de résultats de quatre essais à blanc (sans hydrolysat sec), menés en parallèle.

Ce test F constitue une variante du test de détermination des "fibres alimentaires totales" dans les aliments décrits dans "J. Assoc. Off. Anal. Chem." Vol 68, N° 2, 1985, p 399.

Il présente l'avantage d'être standardisé, de pouvoir être effectué à l'aide d'un kit complet d'analyse, d'être répétable et reproductible.

Ceci étant, on peut également caractériser la digestibilité des polysaccharides obtenus à l'aide du procédé conforme à l'invention en déterminant la teneur en polysaccharides précipitables dans l'éthanol et non hydrolysables par l'amyloglucosidase dans un autre test dénommé A.

Afin de déterminer cette teneur, on s'y prend comme suit.

Un échantillon de 10 g d'hydrolysat de dextrine éventuellement hydrogéné et éventuellement enrichi, par exemple par chromatographie, est amené à un Brix de 75 ± 0,2, soit un indice de réfraction proche de 1,478 avant d'être utilisé pour la détermination du taux de polysaccharides précipitables dans l'éthanol.

L'échantillon de 10 g d'H.A.H à 75 Brix est additionné de 30 $cm^3$ d'eau distillée et de 60 $cm^3$ d'éthanol absolu. Le mélange est laissé reposer pendant 1 heure à 0°C. Il est ensuite centrifugé à 0°C pendant 15 minutes à 10 000 g.

Le culot obtenu est séché en étuve sous vide maintenue à 80°C.

Le poids de précipité obtenu, $p_1$, représente le poids de polysaccharides précipitables dans l'éthanol contenu dans les 10 g d'échantillon de départ, soit environ 7,5 g de matière sèche.

Afin de déterminer la teneur de l'hydrolysat en polysaccharides précipitables dans l'éthanol et non hydrolysables sables par l'amyloglucosidase, on utilise un test A, qui consiste à faire subir aux polysaccharides précipités dans l'éthanol obtenus précédemment une attaque enzymatique à l'aide d'une α-amylase thermorésistante, d'une protéase et d'une amyloglucosidase, puis à opérer une précipitation des polysaccharides non hydrolysables avec de l'éthanol à 95 %, à filtrer le précipité ainsi obtenu, à laver ce dernier plusieurs fois à l'alcool et à l'acétone, et enfin à déterminer le poids, $p_2$, de résidu obtenu.

Ce test est décrit dans "J. Assoc. Off. Anal. Chem." vol. 68, n° 2, 1985, p. 399 auquel on pourra se référer.

La fraction de polysaccharides non digestibles par l'amyloglucosidase se trouve très nettement augmentée par rapport à la teneur en polysaccharides de hauts poids moléculaires lorsque le traitement enzymatique de la même dextrine est effectuée en présence d'enzyme hydrolysant les liaisons 1-6 de l'amylopectine, en comparaison à un traitement sans emploi de cette enzyme. Ceci constitue bien évidemment un avantage essentiel de l'invention, les polysaccharides, hydrogénés ou non, ainsi obtenus présentant ainsi les qualités optimales de fibres alimentaires solubles.

Les produits susceptibles d'être obtenus grâce au procédé conforme à l'invention peuvent être utilisés en tant que substance de charge faiblement hygroscopique et faiblement calorique, support de séchage ou substitut de sucre, de matières grasses, de polyols ou d'autres ingrédients dans tous types d'applications alimentaires, pharmaceutiques ou diététiques, seuls ou en combinaison avec d'autres produits.

Ils présentent en effet une excellente compatibilité avec la très grande majorité des ingrédients alimentaires, pharmaceutiques ou diététiques et peuvent éventuellement être prémélangés sans aucun inconvénient à un conservateur, un émulsifiant, un sucre, un polyol tel que le xylitol, l'érythritol, le mannitol, le sorbitol et le maltitol, un arôme, un édulcorant intense, un acide, un acide aminé, une protéine, une matière grasse, un agent de charge minéral ou organique tels les sels de sodium, de potassium, de calcium, les polydextroses, les fibres, les fructooligosaccharides, les gommes, à un agent gélifiant organique ou minéral tels que les pectines, les celluloses, les extraits d'algues et de graines, les polysaccharides bactériens, à un agent levant, une vitamine, un principe pharmaceutique ou vétérinaire.

En raison de leur grande solubilité dans l'eau, de leurs caractéristiques organoleptiques particulières, de leur excellente stabilité vis-à-vis de la chaleur, vis-à-vis de l'humidité ou des autres ingrédients de formulation, et enfin de leurs propriétés métaboliques remarquables, dont en particulier leur très faible digestibilité, les produits susceptibles d'être obtenus grâce au procédé conforme à l'invention, peuvent être avantageusement utilisés, sous forme d'un sirop ou d'une poudre, dans la préparation de produits de confiserie et de chocolaterie comme par exemple les chewing-gums, les bonbons, les barres céréalières et les chocolats allégés, dans la préparation de produits lactés comme les flans, les yaourts, les mousses et les crèmes dessert, dans la préparation de produits extrudés de type snacks ou céréales pour petit déjeuner, dans la préparation de produits glacés ou surgelés tels que les sorbets, les plats cuisinés, dans la préparation de produits de charcuterie comme les terrines, dans celle de produits issus de la transformation des céréales ou des fruits tels que les nouilles, les biscottes, les cakes, les confitures et les entremets, et également dans la préparation de boissons et de sirops alimentaires, diététiques ou pharmaceutiques.

De plus, en raison de leur très grande stabilité aux enzymes de microorganismes, les produits susceptibles d'être obtenus grâce au procédé conforme à l'invention peuvent également être utilisés en cosmétique comme par exemple dans la formulation de fards à paupières, ou être incorporés dans des produits industriels après avoir été éventuellement modifiés chimiquement ou thermiquement.

## EXEMPLES DE REALISATION DU PROCEDE CONFORME A L'INVENTION

### EXEMPLE 1

Dans une cuve de 25 litres agitée et thermostatée, on introduit 20 litres d'un sirop formé par dilution dans l'eau, à une matière sèche de 30 %, de dextrine jaune TACKIDEX DF 165 commercialisée par la Société Demanderesse.

On ajuste le pH de ce sirop à 5,5 et la température à 55°C, puis on introduit 0,15 °/°° (poids/poids sec) de β-amylase SPEZYME DBA de GENENCOR et 2 °/°° (poids/poids sec) de pullulanase PULLUZYME 750 L de ABM.

Après 72 heures, on acidifie à pH 3,5 et on chauffe la cuve à 80°C pendant 20 minutes pour inhiber les enzymes.

Ce sirop est ensuite filtré puis déminéralisé sur résine cationique forte et anionique faible, puis il subit un traitement à l'eau oxygénée (10 °/°° volume/volume) à l'aide d'une solution d'$H_2O_2$ à 35 % (v/v) pendant 24 heures à 70°C et pH 9,5. On y ajoute ensuite un peu de catalase pour décomposer l'eau oxygénée excédentaire et après dégazage sous vide, on traite ce sirop au charbon actif puis sur un lit de résines mélangées avant de le concentrer à une matière sèche de 75 %.

Ce sirop présente une teneur en polysaccharides non digestibles à l'amyloglucosidase selon le test F de 16,2 %, un précipité à l'alcool $p_1$ selon le test A de 38 % et un précipité $p_2$ de 7,6 % selon le même test, et présente des caractères organoleptiques tout à fait acceptables.

EXEMPLE 2

Dans une cuve de 25 litres agitée et thermostatée, on introduit 20 litres d'un sirop formé par dilution dans l'eau, à une matière sèche de 35 %, de dextrine jaune TACKIDEX DF 165. On ajuste le pH de ce sirop à 5,5 et la température à 55°C, puis on introduit :

Hydrolysats 2a et 2b : 1,5 °/°° (p/p sec) de β-amylase Spezyme DBA de Genencor et 2 °/°° (p/p sec) de pullulanase. Pulluzyme 750 L d'ABM.

Hydrolysat 2c (selon : 1,5 °/°° (p/p sec) de β-amylase l'art antérieur) Spezyme DBA de Genencor.

Après 24 heures, on ajoute aux hydrolysats 2b et 2c 1 °/°° (p/p sec) d'α-amylase Maxamyl HT 3000 de Gist.

Après 86 heures, on acidifie à pH 3,5 et on chauffe la cuve à 80°C pendant 20 minutes pour inhiber les enzymes. Ces sirops sont ensuite filtrés puis déminéralisés sur résines cationique forte et anionique faible et enfin concentrés à 75 % de M.S.

Ces sirops présentent les spectres glucidiques données dans le tableau I.

L'hydrolysat 2a est utilisé pour préparer un sirop hydrogéné. Pour cela, sa matière sèche est amenée à 40 %. On ajoute par rapport au sirop 5 % de catalyseur au nickel de Raney. L'hydrogénation est conduite à une température de 130°C sous une pression d'hydrogène de 50 bars. Elle est poursuivie jusqu'à l'obtention d'un taux de sucres réducteurs inférieur à 0,5 %.

L'hydrolysat ainsi obtenu (hydrolysat 2aH) est ensuite purifié et concentré à 75 % de M.S. Il contient environ 1 % de sorbitol, 22 % de maltitol et d'isomaltitol et 25 % par rapport à la matière sèche de polysaccharides non hydrolysables, selon le test F.

TABLEAU I

| COMPOSITION EN % | EXEMPLES SELON L'INVENTION | | EXEMPLE COMPARATIF HYDROLYSAT 2c |
|---|---|---|---|
| | Hydrolysat 2a | Hydrolysat 2b | |
| DP1 | 0,7 | 1,3 | 1,1 |
| DP2 | 22,3 | 22,0 | 19,3 |
| DP3 | 6,1 | 6,2 | 5,0 |
| DP4 | 3,8 | 3,5 | 3,3 |
| DP5 | 4,3 | 4,3 | 4,7 |
| DP6 | 3,6 | 4,6 | 4,3 |
| DP7 | 4,0 | 3,9 | 3,8 |
| DP 8 à 20 | 28,2 | 28,8 | 28,6 |
| DP > 20 | 27,0 | 28,4 | 29,9 |
| Valeur F en % (teneur selon le test F en polysaccharides non hydrolysables à l'amyloglucosidase) | 26,1 | 23,8 | 27,2 |
| $\dfrac{\text{Valeur F}}{\text{DP > 20}}$ en % | 96,7 | 93,7 | 90,9 |

Les compositions selon l'invention présentent une teneur en maltose plus élevée que la composition de l'art antérieur.

De plus, elles présentent des teneurs en polysaccharides non hydrolysables à l'amyloglucosidase plus faibles par rapport aux matières sèches des hydrolysats, mais augmentées par rapport aux quantités de polysaccharides de hauts poids moléculaires des hydrolysats.

EXEMPLE 3

Dans une cuve de 25 litres agitée et thermostatée, on introduit 20 litres d'un sirop formé par dilution dans l'eau, à une matière sèche de 35 %, de dextrine jaune TACKIDEX DF 165. On ajuste le pH de ce sirop à 5,5 et la température à 55°C, puis on introduit :

| | |
|---|---|
| Hydrolysat 3 a : | 1,5 °/°° (p/p sec) d'amyloglucosidase Amigase TS 300 de GIST, 2 °/°° (p/p sec) de pullulanase Pulluzyme 750 L d'ABM et 1,5 °/°° (p/p sec) d'α-amylase Maxamyl HT 3000 de GIST. |
| Hydrolysat 3b : | 1,5 °/°° (p/p sec) d'amyloglucosidase Amigase TS 300 et 2 °/°° (p/p sec) de pullulanase Pulluzyme 750 L d'ABM. |
| Hydrolysat 3c (selon : l'art antérieur) | 1 °/°° (p/p sec) d'α-amylase fongique MKC LF 40 de Miles. |

Après 86 heures, on acidifie à pH = 3,5 et on chauffe la cuve à 80°C pendant 20 minutes pour inhiber les enzymes.

Ces sirops sont ensuite filtrés, déminéralisés sur résines cationique forte et anionique faible et concentrés à 75 % de M.S. Une partie de l'hydrolysat 3a est hydrogéné comme décrit dans l'exemple 2 (hydrolysat 3aH). L'hydrolysat 3aH contient environ 36,5 % de sorbitol.

Les spectres glucidiques des hydrolysats 3a, 3b et 3c figurent dans le tableau II suivant :

TABLEAU II

| COMPOSITIONS EN % | EXEMPLES SELON L'INVENTION | | EXEMPLE COMPARATIF 3c |
|---|---|---|---|
| | 3a | 3b | |
| DP1 | 35,3 | 34,5 | 32,4 |
| DP2 à DP20 | 46,3 | 46,2 | 41,7 |
| DP > 20 | 18,4 | 19,3 | 25,9 |

Les produits selon l'invention sont plus riches en glucose et plus pauvres en polysaccharides que l'hydrolysat selon l'art antérieur.

Les hydrolysats 3b et 3c sont comparés entre eux à 10 % de M.S. par un jury de dégustation selon un test triangulaire. Les différences entre les deux produits ne sont pas significatives.

Les deux produits sont jugés neutres en goût, peu sucrés et non amers. L'hydrolysat 3aH présente également d'excellentes propriétés organoleptiques.

EXEMPLE 4

Dans une cuve de 25 litres, agitée et thermostatée, on introduit 20 litres d'un sirop formé par dilution dans l'eau, à une matière sèche de 35 %, de dextrine jaune de marque TACKIDEX DF 165.

On ajuste le pH de ce sirop à 5,5 et la température à 55°C puis on introduit :

- hydrolysat 4a

  1,5 °/°° (p/p sec) d'amyloglucosidase Amigase TS 300 de GIST
  2,0 °/°° (p/p sec) de pullulanase Pulluzyme 750 L d'ABM et
  1,5 °/°° (p/p sec) d'α-amylase Maxamyl HT 3000 de GIST.

- Hydrolysat 4b (selon l'art antérieur)
  1,0 % (p/p sec) d'α-amylase fongique MKC LF40 de Miles.

Après 86 heures, on acidifie à pH = 3,5 et on élève la température de la cuve à 80°C pendant 20 minutes pour

8

inhiber les enzymes.

Les deux hydrolysats sont ensuite filtrés, puis chromatographiés sur une résine cationique sodique selon les conditions industrielles habituelles.

Les fractions riches en hauts poids moléculaires sont recueillies et sont purifiées et atomisées (poudres 4a et 4b).

La poudre 4a est remise en solution à 40 % de M.S. puis le sirop obtenu est hydrogéné, purifié et atomisé (poudre 4aH).

Compositions

Dans le tableau III sont donnés les spectres glucidiques des produits 4a et 4b.

TABLEAU III

| COMPOSITIONS EN % | Poudre 4a (invention) | Poudre 4b (selon l'art antérieur) |
|---|---|---|
| DP 1 à DP 5 | 10,7 | 7,7 |
| DP 6 et DP 7 | 10,6 | 8,5 |
| DP 8 à DP 20 | 39,4 | 35,2 |
| DP > 20 | 39,3 | 48,6 |
| Valeur F en % (polysaccharides non hydrolysables à l'amyloglucosidase selon le test F) | 47,6 | 46,4 |
| $\dfrac{\text{Valeur F}}{\text{DP} > 20}$ en % | 121 | 95 |

La poudre 4aH contient 44,1 % de polysaccharides non hydrolysables à l'amyloglucosidase selon le test F et une composition en mono, di, tri, oligo et polysaccharides hydrogénés très voisine en pourcentage de la composition de la poudre 4a non hydrogénée.

Propriétés organoleptiques

- Goût

Les poudres 4a et 4aH sont comparées en goût aux polydextroses A, K et Litesse [R] de la société Pfizer. Les dégustations sont effectuées sur des solutions à 20 % de M.S.

| Sirops à 20 % de M.S. | | | | | |
|---|---|---|---|---|---|
| | Poudre 4a | Poudre 4aH | Polydextrose A | Polydextrose K | Litesse [R] |
| Goûts | Neutre<br>Pas sucré<br>Pas amer<br>Pas d'arrière goût | Neutre<br>Pas sucré<br>Pas amer<br>Pas d'arrière goût | Très acide<br><br>Amer<br>Présence d'un arrière goût | Très faiblement sucré et salé<br>Amer<br>Présence d'un arrière goût | Très faiblement sucré<br>Légère amertume<br>Léger arrière goût |

Les produits selon le procédé conforme à l'invention sont jugés excellents en goût. Ils sont neutres et peuvent de ce fait être employés dans des aliments non sucrés, mais également dans des aliments sucrés par ajout d'un édulcorant de masse (sucre, polyols) ou intense (aspartame, alitame, acésulfame).

- Couleurs en solution

Les différentes solutions à 20 % de M.S. sont concentrées par cuisson jusqu'à 70 % de M.S.

Les solutions sont :

. très légèrement colorées pour les sirops 4aH et 4a
. jaune clair pour le sirop de polydextrose A
. orangée pour le sirop Litesse®
. orange foncé pour le sirop de polydextrose K

Si l'on compare les sirops 4a et 4aH, on constate que l'hydrogénation n'a eu qu'un très léger effet bénéfique. Les produits 4a et 4aH, neutres en couleur, peuvent convenir à la préparation des boissons alimentaires ou diététiques.

Propriétés physico-chimiques

- viscosité

Les sirops 4a et 4aH à 70 % de M.S. présentent respectivement une viscosité de 14,5 et 12,2 Pa.s (14 500 et 12 200 cps).

Ils sont plus visqueux que les sirops de polydextrose à même matière sèche (1,2 à 1,6 Pa.s [1 200 à 1 600 cps]). Les poudres 4a et 4aH sont d'excellents agents viscosifiants et épaississants. Elles peuvent être utilisées de ce fait pour contrôler la cristallisation d'autres produits.

La viscosité des sirops 4a et 4aH peut être abaissée par ajout d'une molécule de faible poids moléculaire (glycérol, sorbitol, xylitol). Ils peuvent alors être plus fortement concentrés (jusqu'à 92 % de matière sèche) et être utilisés comme agents plastifiants et liants.

- Activité de l'eau et hygroscopicité

. A 70 % de M.S., les sirops 4a et 4aH présentent une activité de l'eau voisine de 0,90 contre environ 0,88 pour les sirops de polydextroses.

En d'autres termes, à matière sèche égale, les produits selon l'invention sont moins hygroscopiques et abaissent moins fortement les points de congélation de l'eau.

L'addition d'un sel, d'un sucre ou d'un polyol permet aisément d'ajuster ces valeurs d'activité de l'eau, ce qui permet d'employer ces sirops dans la confection de glaces, de desserts glacés, de produits surgelés et de produits de boulangerie, de biscuiterie et de pâtisserie.

Les poudres 4a et 4aH conservent le même état pulvérulent au cours du stockage que celui des poudres fraîchement atomisées. Elles sont particulièrement peu hygroscopiques et peuvent de ce fait convenir comme support de séchage par exemple de vitamines, d'arômes, de colorant, d'édulcorants intenses ou comme agent de poudrage, agent anticollage, agent antimottant ou agent de gommage.

- Stabilité à la cuisson

Les sirops 4a, 4aH et Litesse® sont concentrés afin de fabriquer des sucres cuits par cuisson à 130°C. Le produit final obtenu avec le sirop 4aH est de loin le moins coloré. Les produits conformes à l'invention sont très stables, en particulier le produit 4aH hydrogéné. Ceci est particulièrement intéressant et permet d'utiliser ces sirops pour la confection de produits cuits comme les articles de confiserie.

- Stabilité aux enzymes

Les sirops 4a et 4aH, en particulier le sirop 4aH, sont plus stables que les polydextroses vis-à-vis des enzymes présentes dans la cavité buccale.

Le sirop 4aH n'est pas cariogène.

EXEMPLE 5 : CHEWING-GUMS FAIBLEMENT CALORIQUES ET SANS SUCRE CONTENANT DES POLYSACCHARIDES SOLUBLES INDIGESTIBLES

On prépare des chewing-gums de type bubble-gum contenant des polysaccharides faiblement digestibles obtenus par le procédé selon l'invention.

On suit pour cela le mode opératoire suivant, permettant d'obtenir 3 kg de pâte de chewing-gum.

On prépare d'abord deux prémélanges édulcorants :

- le premier contient 585 g de poudre de sorbitol Neosorb® P 100 T commercialisé par la Société Demanderesse, 585 g de poudre 4aH selon l'invention, 27 g d'acide malique broyé et 9 g d'acide citrique broyé.
- le second contient 270 g de xylitol Xylisorb® commercialisé par la Société Demanderesse, 10 g d'aspartame, 100 g d'érythritol broyé, 160 g de poudre 4aH selon l'invention et 24 g d'arôme d'orange.

Par la suite, on introduit dans un pétrin de type bras en "Z" chauffé à 50°C, 780 g de gomme de base bubble-gum, préalablement ramollie (provenant de la Société Cofosa).

On ajoute ensuite la moitié de la quantité du premier prémélange en continuant à pétrir pendant 2 mn. On introduit ensuite 225 g d'hydrolysat 2aH.

Après une minute de pétrissage, on ajoute la seconde moitié du premier prémélange, on pétrit à nouveau 2 mn avant d'incorporer 225 g de sirop de maltitol Lycasin® 80/55 à 85 % de M.S., commercialisé par la Société Demanderesse.

Enfin, on ajoute après deux minutes supplémentaires de mélange le second prémélange. L'ensemble est malaxé trois minutes supplémentaires.

Après quoi, on lamine et on forme la pâte de chewing-gum obtenue en utilisant comme poudre de talcage peu hygroscopique un mélange à base de 6,9 g de mannitol, 3,0 g de la poudre 4aH selon l'invention et 0,1 g d'acésulfame K.

On obtient de la sorte des chewing-gums souples et peu collants et de très bon goût, contenant environ 12 % de polysaccharides indigestibles.

Au cours du stockage, ces chewing-gums restent souples et n'ont pas tendance à reprendre de l'eau. Les polysaccharides solubles, indigestibles, incorporés dans ces chewing-gums jouent le rôle d'un anticristallisant peu hygroscopique. Ils peuvent de ce fait convenir à tous types de chewing-gums, qu'ils contiennent ou non de l'eau.

## Revendications

1. Procédé de préparation de polysaccharides peu digestibles, caractérisé par le fait qu'il comprend l'hydrolyse enzymatique d'au moins une dextrine et/ou d'un polyglucose à l'aide d'au moins une enzyme saccharifiante et d'au moins une enzyme hydrolysant les liaisons 1-6 de l'amylopectine.

2. Procédé selon la revendication 1, caractérisé par le fait que les conditions de l'hydrolyse enzymatique sont choisies de façon telle que le dextrose-équivalent (DE) de l'hydrolysat obtenu soit compris entre 5 et 80, de préférence entre 7 et 60, et plus préférentiellement encore entre 10 et 50.

3. Procédé selon l'une ou l'autre des revendications 1 et 2, caractérisé par le fait que l'enzyme saccharifiante est choisie parmi la bêta-amylase et l'amyloglucosidase, ces deux enzymes pouvant éventuellement être utilisées de façon simultanée ou successive.

4. Procédé selon l'une quelconque des revendications 1 à 3, caractérisé par le fait que l'enzyme hydrolysant les liaisons 1-6 de l'amylopectine est une isoamylase ou une pullulanase.

5. Procédé selon l'une quelconque des revendications 1 à 4, caractérisé par le fait que l'hydrolyse enzymatique de la dextrine et/ou du polyglucose est précédée, accompagnée ou suivie d'une action complémentaire à l'aide d'une alpha-amylase.

6. Procédé selon l'une quelconque des revendications 1 à 5, caractérisé par le fait que l'hydrolyse enzymatique de la dextrine et/ou du polyglucose est précédée d'une hydrolyse acide.

7. Procédé selon l'une quelconque des revendications 1 à 6, caractérisé par le fait que les conditions d'action des différentes enzymes sont choisies parmi les suivantes :

   - Bêta-amylase : 1,45 à 145 unités internationales (100 à 10 000 unités LINTNER) par kg de substrat sec, température de 50°C à 60°C, durée d'action de 30 à 100 heures, pH de 5,0 à 6,0 ;
   - amyloglucosidase : 4 000 à 500 000 unités internationales par kg de substrat sec, température de 50°C à 60°C, durée d'action de 30 à 100 heures, pH de 5,0 à 6,0 ;
   - enzyme hydrolysant les liaisons 1-6 de l'amylopectine : 435 à 43 500 unités internationales (150 à 15 000 unités ABM) par kg de substrat sec, pH de 5,0 à 6,0, température de 50°C à 60°C, durée d'action de 24 à 100 heures ;
   - alpha-amylase ; 0,35 à 35 unités internationales (20 à 2 000 unités KiloNovo Units) par kg de substrat sec, pH de 5,0 à 6,0, température de 50°C à 60°C, durée d'action de 16 à 100 heures.

8. Procédé selon l'une quelconque des revendications 1 à 7, caractérisé par le fait que l'on hydrogène le produit obtenu à la suite de l'hydrolyse enzymatique de la dextrine et/ou du polyglucose.

9. Procédé selon la revendication 8, caractérisé par le fait que l'hydrogénation est conduite jusqu'à obtention d'un pourcentage de sucres réducteurs résiduels sur matière sèche inférieur à 2,0, de préférence inférieur à 1,0 et plus préférentiellement encore inférieur à 0,25.

10. Polysaccharides peu digestibles susceptibles d'être obtenus par le procédé selon l'une quelconque des revendications 1 à 9.

11. Utilisation des polysaccharides susceptibles d'être obtenus par le procédé selon l'une quelconque des revendications 1 à 9 dans la préparation des produits alimentaires, pharmaceutiques ou diététiques.

12. Utilisation des polysaccharides susceptibles d'être obtenus par le procédé selon l'une quelconque des revendication 1 à 9 dans la préparation des produits de type chewing-gum.

**Patentansprüche**

1. Verfahren zur Herstellung von schwer verdaulichen Polysacchariden, dadurch gekennzeichnet, daß es die enzymatische Hydrolyse von mindestens einem Dextrin und/oder einer Polyglucose mit Hilfe von mindestens einem verzuckernden Enzym und mindestens einem die Bindungen 1-6 des Amylopectins hydrolysierenden Enzym umfaßt.

2. Verfahren nach Anspruch 1, dadurch gekennzeichnet, daß die Bedingungen der enzymatischen Hydrolyse in der Weise gewählt werden, daß das Dextrose-Äquivalent (DE) des erhaltenen Hydrolysates zwischen 5 und 80, vorzugsweise zwischen 7 und 60 und ganz besonders bevorzugt zwischen 10 und 50 beträgt.

3. Verfahren nach dem einen oder anderen der Ansprüche 1 und 2, dadurch gekennzeichnet, daß das verzuckernde Enzym unter beta-Amylase und Amyloglucosidase ausgewählt wird, wobei diese beiden Enzyme gegebenenfalls gleichzeitig oder nacheinander verwendet werden.

4. Verfahren nach irgendeinem der Ansprüche 1 bis 3, dadurch gekennzeichnet, daß das die Bindungen 1-6 des Amylopectins hydrolysierende Enzym eine Isoamylase oder eine Pullulanase ist.

5. Verfahren nach irgendeinem der Ansprüche 1 bis 4, dadurch gekennzeichnet, daß der enzymatischen Hydrolyse des Dextrins und/ oder der Polyglucose eine zusätzliche Einwirkung mit Hilfe einer alpha-Amylase vorangeht, die Hydrolyse von ihr begleitet wird oder sie der Hydrolyse nachfolgt.

6. Verfahren nach irgendeinem der Ansprüche 1 bis 5, dadurch gekennzeichnet, daß der enzymatischen Hydrolyse des Dextrins und/ oder der Polyglucose eine saure Hydrolyse vorangeht.

7. Verfahren nach irgendeinem der Ansprüche 1 bis 6, dadurch gekennzeichnet, daß die Bedingungen zur Einwirkung der verschiedenen Enzyme unter den folgenden gewählt werden:

   - beta-Amylase: 1,45 bis 145 Internationale Einheiten (100 bis 10.000 Einheiten LINTNER) pro kg trockenes Substrat, Temperatur 50 °C bis 60 °C, Dauer der Einwirkung 30 bis 100 Stunden, pH-Wert 5,0 bis 6,0;
   - Amyloglucosidase: 4.000 bis 500.000 Internationale Einheiten pro kg trockenes Substrat, Temperatur 50 °C bis 60 °C, Dauer der Einwirkung 30 bis 100 Stunden, pH-Wert 5,0 bis 6,0;
   - die Bindungen 1-6 des Amylopectins hydrolysierendes Enzym: 435 bis 43.500 Internationale Einheiten (150 bis 15.000 Einheiten ABM) pro kg trockenes Substrat, pH-Wert 5,0 bis 6,0; Temperatur 50 °C bis 60 °C, Dauer der Einwirkung 24 bis 100 Stunden;
   - alpha-Amylase: 0,35 bis 35 Internationale Einheiten (20 bis 2.000 Einheiten KiloNovo Units) pro kg trockenes Substrat, pH-Wert 5,0 bis 6,0; Temperatur 50 °C bis 60 °C, Dauer der Einwirkung 16 bis 100 Stunden.

8. Verfahren nach irgendeinem der Ansprüche 1 bis 7, dadurch gekennzeichnet, daß man das nach der enzymatischen Hydrolyse des Dextrins und/oder der Polyglucose erhaltene Produkt hydriert.

9. Verfahren nach Anspruch 8, dadurch gekennzeichnet, daß die Hydrierung bis zum Erreichen eines Prozentsatzes an in der Trockensubstanz verbleibenden reduzierenden Zuckern von unter 2,0, vorzugsweise von unter 1,0 und ganz besonders bevorzugt von unter 0,25 durchgeführt wird.

10. Schwer verdauliche Polysaccharide, die geeignet sind, gemäß dem Verfahren nach irgendeinem der Ansprüche 1 bis 9 erhalten zu werden.

11. Verwendung der Polysaccharide, die geeignet sind, gemäß dem Verfahren nach irgendeinem der Ansprüche 1 bis 9 erhalten zu werden, zur Herstellung von Ernährungsprodukten, pharmazeutischen oder diätetischen Produkten.

12. Verwendung der Polysaccharide, die geeignet sind, gemäß dem Verfahren nach irgendeinem der Ansprüche 1 bis 9 erhalten zu werden, zur Herstellung von Produkten des Typs Kaugummi.

**Claims**

1. A process for preparing indigestible polysaccharides characterized in that it comprises the enzymatic hydrolysis of at least one dextrin and/or one polyglucose using at least one saccharifying enzyme and at least one enzyme which hydrolyzes the 1-6 bonds of amylopectin.

2. The process as claimed in claim 1, characterized in that the conditions for the enzymatic hydrolysis are chosen such that the dextrose equivalent (DE) of the hydrolysate obtained is between 5 and 80, preferably between 7 and 60, and still more preferably between 10 and 50.

3. The process as claimed in either of claims 1 and 2, characterized in that the saccharifying enzyme is chosen from β-amylase and amyloglucosidase, it being possible for these two enzymes to be used, where appropriate, simultaneously or successively.

4. The process as claimed in any one of claims 1 to 3, characterized in that the enzyme which hydrolyzes the 1-6 bonds of amylopectin is an isoamylase or a pullulanase.

5. The process as claimed in any one of claims 1 to 4, characterized in that the enzymatic hydrolysis of dextrin and/or polyglucose is preceded, accompanied or followed by an additional action using an α-amylase.

6. The process as claimed in any one of claims 1 to 5, characterized in that the enzymatic hydrolysis of the dextrin and/or the polyglucose is preceded by an acid hydrolysis.

7. The process as claimed in any one of claims 1 to 6, wherein the conditions for the action of the various enzymes are chosen from the following :

   - β-amylase : 1.45 to 145 international units (100 to 10,000 LINTNER units) per kg of dry substrate, temperature of 50°C to 60°C, duration of action from 30 to 100 hours, pH of 5.0 to 6.0 ;
   - amyloglucosidase : 4,000 to 500,000 international units per kg of dry substrate, temperature of 50°C to 60°C, duration of action from 30 to 100 hours, pH of 5.0 to 6.0 ;
   - enzyme hydrolyzing the 1-6 bonds of amylopectin : 435 to 43,500 international units (150 to 15,000 ABM units) per kg of dry substrate, pH of 5.0 to 6.0, temperature of 50°C to 60°C, duration of action from 24 to 100 hours ;
   - α-amylase : 0.35 to 35 international units (20 to 2,000 Kilo Novo Units) per kg of dry substrate, pH of 5.0 to 6.0, temperature of 50°C to 60°C, duration of action from 16 to 100 hours.

8. The process as claimed in any one of claims 1 to 7, characterized in that the product obtained following the enzymatic hydrolysis of dextrin and/or polyglucose is hydrogenated.

9. The process as claimed in claim 8, characterized in that the hydrogenation is carried out until a percentage ratio of residual sugars to dry matter of less than 2.0, preferably less than 1.0 and still more preferably less than 0.25, is obtained.

10. Polysaccharides indigestible apt to be obtained by the process as claimed in any one of claims 1 to 9.

11. Use of the polysaccharides obtained by the process as claimed in any one of claims 1 to 9, in the preparation of food, pharmaceutical or dietary products.

12. Use of the polysaccharides obtained by the process as claimed in any one of claims 1 to 9, in the preparation of chewing gum type products.